# EUROPEAN PATENT SPECIFICATION

(11) **EP 3 182 956 B1**
(45) Date of publication and mention of the grant of the patent: **19.12.2018**
(21) Application number: 14781697.9
(22) Date of filing: 18.08.2014
(51) Int. Cl.: A61K 8/44, A61Q 11/00, A61K 8/22, A61K 8/34, A61K 8/60, A61K 8/86

(54) **MOUTHWASH COMPOSITION COMPRISING A PEROXIDE SOURCE AND AN N-ACYL-L-ARGININE ALKYL ESTER SALT**
MUNDWASSERZUSAMMENSETZUNG MIT EINER PEROXIDQUELLE UND EINEM N-ACYL-L-ARGININ-ALKYLESTERSALZ
COMPOSITION POUR BAIN DE BOUCHE COMPRENANT UNE SOURCE DE PEROXYDE ET UN SEL D'ESTER N-ACYL-L-ARGININE ALKYLIQUE

(43) Date of publication of application: 28.06.2017
(73) Proprietor: Colgate-Palmolive Company, New York, NY 10022 (US)
(72) Inventor: AHMED, Rabab, Somerset, New Jersey 08873 (US); MILLER, Jeffrey, Jackson, New Jersey 08527 (US); PRENCIPE, Michael, West Windsor, New Jersey 08550 (US); PICQUET, Guillaume, Piscataway, NJ 08854 (US)
(74) Representative: Wibbelmann, Jobst
(86) International application number: PCT/US2014/051562
(87) International publication number: WO 2016/028265

(56) References cited:
- WO-A1-2014/025355
- US-A- 5 874 068
- US-A1- 2005 027 001
- US-A1- 2007 014 740

## Description

### BACKGROUND

Dental plaque is a soft deposit which forms on teeth and comprises an accumulation of bacteria and bacterial by-products. Besides being unsightly, plaque is implicated in the occurrence of gingivitis and other forms of periodontal disease. Cationic anti-bacterial agents (for example, arginine-based esters, Sn (II) compounds, cetylpyridinium chloride) have been proposed for use in oral care, and in particular, to counter plaque formation and oral infections associated with plaque formation. However, these agents are often incompatible with other ingredients found in mouthwash formulations reducing their anti-microbial activity in such formulations.

Peroxide compounds (for example, hydrogen peroxide (H₂O₂)) can provide whitening benefit in mouthwash formulations. However, these formulations potentially lose their whitening efficacy over time as peroxide compounds in aqueous solutions are relatively unstable. Furthermore, peroxide compounds are incompatible with many active oral care agents. In particular, many cationic anti-bacterial agents promote the instability of peroxide compounds and/or are themselves unstable in the presence of peroxide compounds. For example, SnF₂ (Sn II) has anti-bacterial activity in the absence of peroxide compounds. However, SnF₂ is unstable in the presence of peroxides and undergoes oxidation to Sn (IV). Other cationic antibacterial agents such as cetylpyridinium chloride (CPC) stain dentition and therefore reduce the whitening efficacy of peroxide compounds.

Document WO2014/025355 discloses mouthwash compositions comprising peroxy compounds stabilized by acids. Documents US-A-5874068 and US2005/0027001 provide antiplaque and antigingivitis stable oral care compositions based on N-acyl amino acid ester salts.

Therefore, it would be highly desirable to provide a mouthwash formulation which has both effective whitening activity and anti-microbial activity, and in which the whitening activity and the anti-microbial activity are maintained over time.

### BRIEF SUMMARY

The present inventors have unexpectedly found that a salt of an N^{α}-acyl-L-arginine alkyl ester, preferably having the structure of Formula 1 below, and a peroxide source, are compatible with each other within an aqueous mouthwash composition, and in combination, provide effective anti-microbial activity and whitening activity which is maintained over time. The present inventors have unexpectedly found that the salt of the N^{α}-acyl-L-arginine alkyl ester does not have any destabilizing effect on the peroxide source in the aqueous mouthwash composition, and moreover, that in the presence of the peroxide source in the aqueous mouthwash composition, the N^{α}-acyl-L-arginine alkyl ester salt retains anti-microbial activity over time.

Accordingly, in a first aspect, there is provided an aqueous mouthwash composition comprising:
(a) a peroxide source, and
(b) an N^{α}-acyl-L-arginine alkyl ester salt

Preferably, the N^{α}-acyl-L-arginine alkyl ester salt is represented by Formula 1: 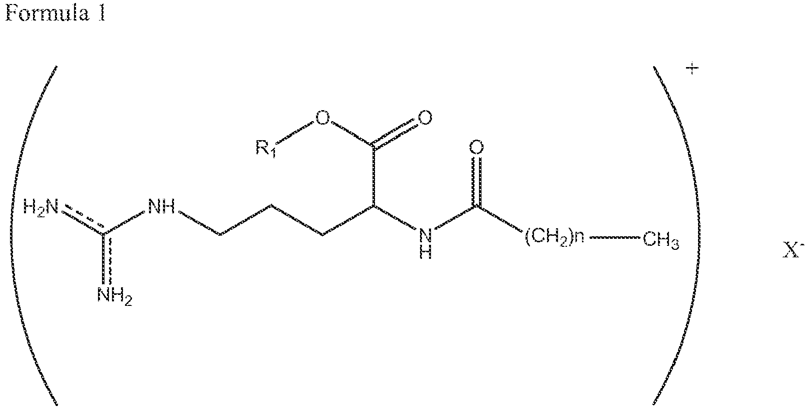 wherein R₁ is an alkyl group having 1 to 6 carbon atoms, n is an integer from 6 to 16, and X⁻ is an anion.

Preferably, the peroxide source is selected from: hydrogen peroxide, urea peroxide, sodium percarbonate, sodium perborate, polyvinylpyrrolidone-hydrogen peroxide complex, and mixtures thereof. More preferably, the peroxide source is hydrogen peroxide.

Optionally, the peroxide source is present in the composition in an amount of 0.1 weight % to 3 weight % by total weight of the composition. Preferably, the peroxide source is present in the composition in an amount of 1 weight % to 2 weight % by total weight of the composition. More preferably, the peroxide source is present in the composition in an amount of 2 weight % by total weight of the composition.

Preferably, R₁ is ethyl and n is 10. More preferably, the N^{α}-acyl-L-arginine alkyl ester salt comprises ethyl lauroyl arginine hydrochloride (N^{α}-lauroyl-L-arginine ethyl ester hydrochloride).

Optionally, the N^{α}-acyl-L-arginine alkyl ester salt is present in the composition in an amount of from 0.05 weight % to 0.4 weight % by total weight of the composition, on an active ingredient basis. Preferably, the N^{α}-acyl-L-arginine alkyl ester salt is present in the composition in an amount of from 0.1 weight % to 0.3 weight % by total weight of the composition, on an active ingredient basis. More preferably, the N^{α}-acyl-L-arginine alkyl ester salt is present in the composition in an amount of 0.2 weight % by total weight of the composition, on an active ingredient basis.

Optionally, the composition further comprises an agent selected from surfactants, humectants, foaming agents, flavorants and sweeteners.

Optionally, the composition further comprises a non-ionic surfactant. Further optionally, the non-ionic surfactant is selected from poloxamers and polyoxyethylene sorbitan esters. Still further optionally, the composition comprises a poloxamer and a polyoxyethylene sorbitan ester.

Optionally, the composition further comprises a humectant. Further optionally, the composition comprises glycerin and/or sorbitol.

Optionally, the composition comprises water in an amount of 70 weight % to 80 weight %, by total weight of the composition. Further optionally, the composition is free of anti-microbial agents other than the N^{α}-acyl-L-arginine ethyl ester salt and the peroxide source. Still further optionally, the composition is a liquid mouthwash composition.

In a second aspect, there is provided an aqueous mouthwash composition for use in inhibiting bacterial attachment to an oral cavity and/or in reducing bacterial viability in an oral cavity, wherein the composition comprises :
(a) a peroxide source, and
(b) an N^{α}-acyl-L-arginine alkyl ester salt.
Preferably, the N^{α}-acyl-L-arginine alkyl ester salt is represented by Formula 1: 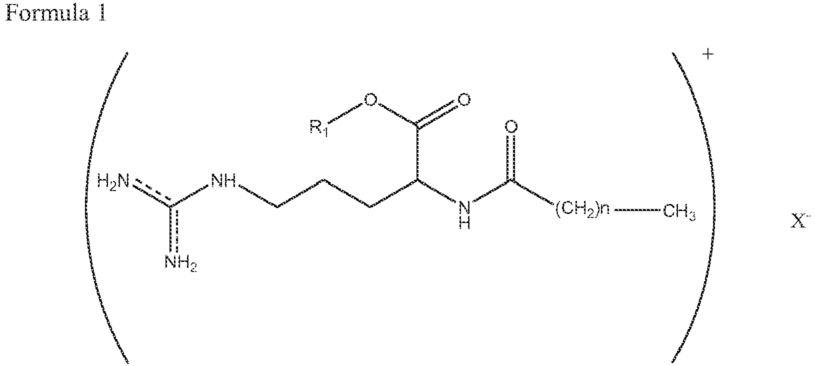 wherein R₁ is an alkyl group having 1 to 6 carbon atoms, n is an integer from 6 to 16, and X⁻ is an anion.

Optionally, the composition is for preventing or treating gingivitis. Preferably, the use further comprises whitening teeth.

Optionally, the composition is as defined herein.

In a third aspect, there is provided a use of an N^{α}-acyl-L-arginine alkyl ester salt in an aqueous mouthwash composition comprising a peroxide source, for maintaining stability of the N^{α}-acyl-L-arginine alkyl ester salt and/or the peroxide source.

Preferably, the N^{α}-acyl-L-arginine alkyl ester salt is represented by Formula 1: 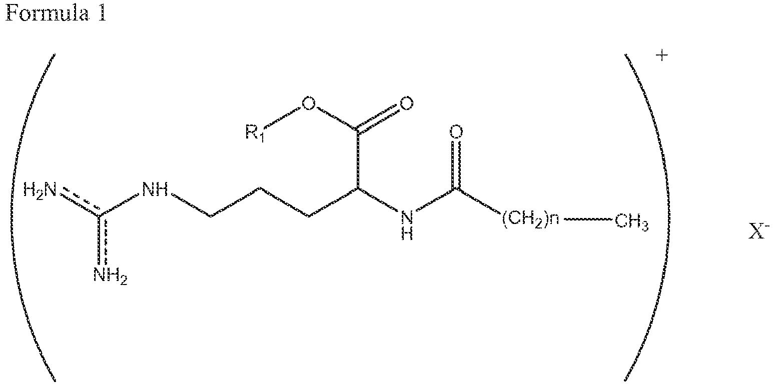 wherein R₁ is an alkyl group having 1 to 6 carbon atoms, n is an integer from 6 to 16, and X⁻ is an anion.

Optionally, the composition is as defined herein.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiments) is merely exemplary in nature and is in no way intended to limit the invention, its application, or uses.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

In one arrangement, provided herein is an aqueous oral care composition comprising:
(a) peroxide source, and
(b) an N^{α}-acyl-L-arginine alkyl ester salt.

The N^{α}-acyl-L-argmine alkyl ester salt may be represented by Formula 1: 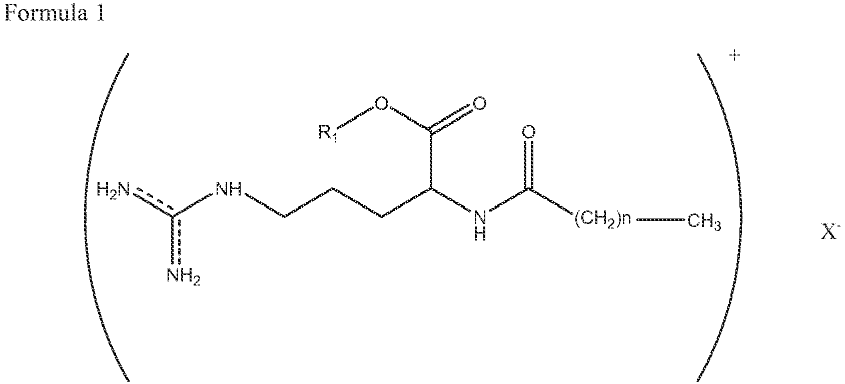 wherein R₁ is an alkyl group having 1 to 6 carbon atoms, n is an integer from 6 to 16, and X⁻ is an anion.

Typically, the oral care composition is a mouthwash or a mouthrinse composition.

### N^{α}-acyl-L-arginine alkyl ester salt

With reference to Formula 1, R₁ may be methyl, ethyl, propyl, butyl, pentyl or hexyl. Preferably, R₁ is ethyl such that the N^{α}-acyl-L-arginine ethyl ester salt is an N^{α}-acyl-L-arginine ethyl ester salt.

In one embodiment, n may be 6, 7, 8, 9, 10, 11, 12, 13, 14, 15 or 16. Preferably, n is an integer from 8 to 12, or from 8 to 10. More preferably, n is 10 such that the N^{α}-acyl-L-arginine ethyl ester salt is an N^{α}-lauroyl-L-argmine alkyl ester salt.

In one embodiment, R₁ is ethyl and n is 10 such that the N^{α}-acyl-L-arginine ethyl ester salt is an N^{α}-lauroyl-L-arginine ethyl ester salt.

In one embodiment, X⁻ may be any counter-anion that provides the arginate compound with a reasonable degree of solubility in water (preferably at least about 1g in 1 liter of water). Examples of counter-anions that may be used include halide ions such as Cl⁻ and Bf, dihydrogen phosphate, acetate, tartrate, citrate, or pyrrolidone-carboxylate. Typically, X⁻ is Cl⁻.

Specific examples of N^{α}-acyl-L-arginine alkyl ester salts that may be used in the present invention include salts, particularly hydrochloride salts, of N^{α}-cocoyl-L-arginine methyl ester, N^{α} -cocoyl-L-arginine ethyl ester, N^{α}-cocoyl-L-arginine propyl ester, N^{α} -stearoyl-Larginine methyl ester, N^{α} - stearoyl-L-arginine ethyl ester, N^{α}-lauroyl-L-arginine methyl ester , N^{α}-lauroyl-L-arginine ethyl ester and N^{α}-lauroyl-L-arginine propyl ester. The term "cocoyl" is an abbreviation for coconut oil fatty acid residue. N^{α}-acyl-L-arginine alkyl ester salts as defined above show excellent inhibitory effects against microorganisms which are involved in plaque formation such as *S. aureus, S. mutans,* and *F. nucleatum.*

In one embodiment, the N^{α}-acyl-L-arginine alkyl ester salt is ethyl lauroyl arginate HCl (N^{α}-lauroyl-L-arginine ethyl ester HCl) according to Formula 2. Ethyl lauroyl arginate HCl is available commercially as Aminat®-G.

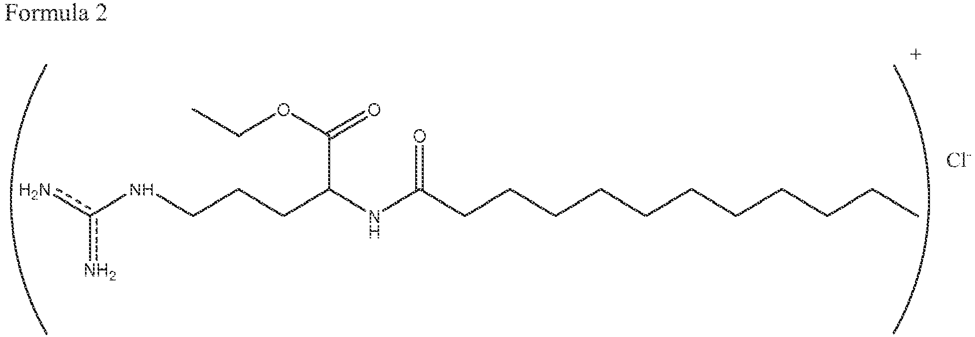

In one embodiment, the N^{α}-acyl-L-argmine alkyl ester salt is present in the composition in an amount of 0.05 weight % to 0.4 weight % by total weight of the composition, on an active ingredient basis. Preferably, the N^{α}-acyl-L-arginine alkyl ester salt is present in the composition in an amount of 0.05 weight % to 0.3 weight %, or from 0.05 weight % to 0.2 weight % by total weight of the composition, on an active ingredient basis. More preferably, the N^{α}-acyl-L-arginine alkyl ester salt is present in the composition in an amount of 0.1 weight % to 0.3 weight %, or from 0.1 weight % to 0.2 weight % by total weight of the composition, on an active ingredient basis. Typically, the N^{α}-acyl-L-arginine alkyl ester salt is present in the composition in an amount of 0.2 weight % by total weight of the composition, on an active ingredient basis.

Cationic anti-bacterial agents, and particularly, salts of N^{α}-acyl-L-arginine alkyl esters (for example, N^{α}-lauroyl-L-arginine ethyl ester salts) are known to be incompatible with many components commonly found in mouthwash or mouthrinse formulations. However, the present inventors have unexpectedly found that in the mouthwash or mouthrinse compositions according to the present invention comprising an N^{α}-acyl-L-arginine alkyl ester salt according to Formula 1 and a peroxide source, the activity and stability of both the N^{α}-acyl-L-arginine alkyl ester salt and the peroxide is maintained in the mouthwash over time.

### Peroxide source

The compositions provided herein comprise a peroxide source. Suitable peroxide sources include hydrogen peroxide, peroxides of alkali and alkaline earth metals, organic peroxide compounds and peroxy acids and salts thereof. The term "peroxide source" includes any orally acceptable compound that delivers a perhydroxyl ion (GOH⁻). A peroxide source can optionally be present in a form of a polymer-peroxide complex, for example a polyvinylpyrrolidone-hydrogen peroxide complex.

Peroxides of alkali and alkaline earth metals include lithium peroxide, potassium peroxide, sodium peroxide, magnesium peroxide, calcium peroxide and barium peroxide. Organic peroxide sources include, for example, carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, monoperoxyphthalate and the like.

Peroxy acids and their salts include organic peroxy acids such as alkyl peroxy acids and monoperoxyphthalate, as well as inorganic peroxy acid salts including persulfate, dipersulfate, percarbonate, perphosphate, perborate and persilicate salts of alkali and alkaline earth metals such as lithium, potassium, sodium, magnesium, calcium and barium. Another useful peroxy compound is sodium pyrophosphate peroxyhydrate.

Typically, the peroxide source is selected from: hydrogen peroxide, urea peroxide, sodium percarbonate, sodium perborate, polyvinylpyrrolidone-hydrogen peroxide complex, and mixtures thereof. In a preferred embodiment, the peroxide source is hydrogen peroxide.

Typically, the peroxide source is present in the composition in an amount of from 0.1 weight % to 3 weight % by total weight of the composition, on an active ingredient basis. Preferably, the peroxide source is present in the composition in an amount of from 0.1 weight % to 2 weight %, or from 0.1 weight % to 1 weight % by total weight of the composition, on an active ingredient basis. In some embodiments, the peroxide source is present in the composition in an amount of 0.5 weight % to 3 weight %, or from 0.5 weight % to 2 weight %, or from 0.5 weight % to 1 weight %, by total weight of the composition, on an active ingredient basis. Preferably, the peroxide source is present in the composition in an amount of from 1 weight % to 3 weight %, or from 1 weight % to 2 weight % by total weight of the composition, on an active ingredient basis. Typically, the peroxide source is present in the composition in an amount of 0.2 weight % by total weight of the composition, on an active ingredient basis.

The compositions of the present invention are typically in the form of mouthwash or mouthrinse formulations, and preferably, aqueous liquid mouthwash or mouthrinse formulations. However, other forms such as sprays are also envisaged. The compositions preferably comprise water in an amount of from 60 weight % to 90 weight % by total weight of the composition. Typically, the compositions comprise water in an amount of from 65 weight % to 85 weight %, or from 70 weight % to 80 weight %, by total weight of the composition.

### Optional Ingredients

The oral care compositions of the present invention may further comprise additional ingredients. These additional ingredients may include, but are not limited to, diluents, pH modifying agents, surfactants, foam modulators, humectants, sweeteners, flavorants, antioxidants, sources of fluoride ions, anti-hypersensitivity agents and mixtures thereof. Such ingredients would be known to those skilled in the art of oral care. However, non-limiting examples of these ingredients are provided below.

### Surfactants

The oral care compositions of the invention may comprise at least one surfactant. Any orally acceptable surfactant, for example, those which are anionic, nonionic or amphoteric, can be used. Suitable anionic surfactants include without limitation, water-soluble salts of C₈₋₂₀ alkyl sulfates, sulfonated monoglycerides of C₈₋₂₀ fatty acids, sarcosinates, taurates and the like. Illustrative examples of these and other classes include sodium lauryl sulfate, sodium coconut monoglyceride sulfonate, sodium lauryl sarcosinate, sodium lauryl isoethionate, sodium laureth carboxylate and sodium dodecyl benzenesulfonate. Suitable amphoteric surfactants include without limitation, derivatives of C₈₋₂₀ aliphatic secondary and tertiary amines having an anionic group such as carboxylate, sulfate, sulfonate, phosphate or phosphonate. Betaines may also be used, a suitable example of which is cocoamidopropyl betaine. Suitable nonionic surfactants include without limitation, poloxamers (for example, Poloxamer 407), polyoxyethylene sorbitan esters (for example, polysorbate 20), fatty alcohol ethoxylates, alkylphenol ethoxylates, tertiary amine oxides, tertiary phosphine oxides, dialkyl sulfoxides and the like.

In one arrangement, the composition comprises a non-ionic surfactant selected from poloxamers and polyoxyethylene sorbitan esters. Preferably, the composition comprises a poloxamer and a polyoxyethylene sorbitan ester.

One or more surfactants are optionally present in a total amount of about 0.01 weight% to about 10 weight %, for example, from about 0.05 weight % to about 5 weight %, or from about 0. 1 weight % to about 2 weight % by total weight of the composition, on an active ingredient basis.

### Humectants

Within certain embodiments of the oral compositions, it is also desirable to incorporate a humectant to reduce evaporation and also contribute towards preservation by lowering water activity. Certain humectants can also impart desirable sweetness or flavor to the compositions. The humectant may be present in the composition in an amount of from 10 weight % to 40 weight % in one embodiment, or from 15 weight % to 30 weight % in another embodiment, by total weight of the composition.

Suitable humectants include edible polyhydric alcohols such as glycerine, sorbitol, xylitol, propylene glycol as well as other polyols and mixtures of these. Typically, the composition of the present invention comprises a combination of glycerine and sorbitol.

### Flavorants

The oral care compositions of the invention may also include a flavoring agent. Flavoring agents which are used in the practice of the present invention include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

One or more flavorants are optionally incorporated in the oral composition at a concentration of 0.01 to 1 weight % by total weight of the composition.

### Fluoride ion source

The oral care compositions may further comprise a fluoride ion source in an amount sufficient to provide from 50 to 5000 ppm, preferably from 100 to 1000 ppm fluoride ions. Fluoride ion sources include, but are not limited to: stannous fluoride, sodium fluoride, potassium fluoride, potassium monofluorophosphate, sodium monofluorophosphate, ammonium monofluorophosphate, sodium fluorosilicate, ammonium fluorosilicate, amine fluoride such as olaflur (N'-octadccyltrimethylendiamine-N,N,N'-tris(2-ethanol)-dihydrofluoride), ammonium fluoride, and combinations thereof.

### Uses

As mentioned above, many cationic anti-bacterial agents promote the instability of peroxide compounds and/or are themselves unstable in the presence of peroxide compounds. For example, SnF₂ (Sn II) has anti-bacterial activity in the absence of peroxide compounds. However, SnF₂ is unstable in the presence of peroxides and undergoes oxidation to Sn (IV). Other cationic antibacterial agents such as cetylpyridinium chloride (CPC) stain dentition and therefore reduce the whitening efficacy of peroxide compounds. The present inventors have unexpectedly found that an N^{α}-acyl-L-arginine alkyl ester salt, preferably according to Formula 1 as defined above, can be effectively combined with a peroxide source in an aqueous mouthwash composition, without any impact on the stability or activity of either agent. Thus, oral care compositions according to the present invention have effective anti-microbial activity and whitening activity. Accordingly, in some embodiments, the compositions are free of anti-microbial agents other than the N^{α}-acyl-L-arginine alkyl ester salt and the peroxide source.

Accordingly, in one arrangement, there is provided an aqueous oral care composition comprising:
(a) a peroxide source, and
(b) an N^{α}-acyl-L-arginine alkyl ester salt.
Typically, the N^{α}-acyl-L-arginine alkyl ester salt is represented by Formula 1: 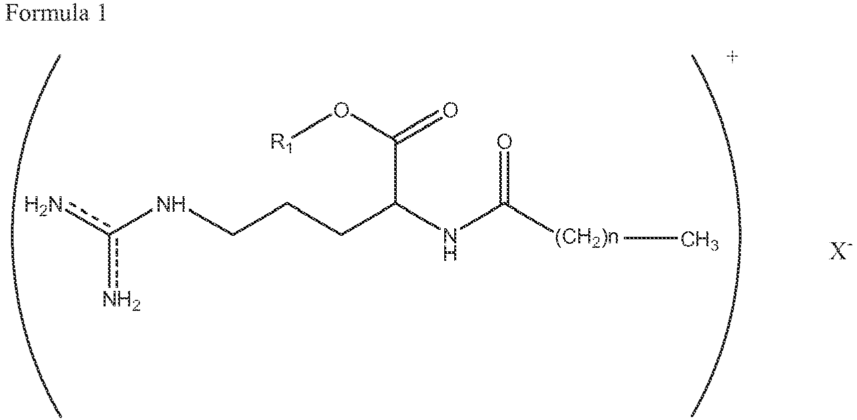 wherein R₁ is an alkyl group having 1 to 6 carbon atoms, n is an integer from 6 to 16, and X⁻ is an anion, for use in inhibiting bacterial attachment to an oral cavity and/or in reducing bacterial viability in an oral cavity. Typically, the composition is a mouthwash or a mouthrinse. Preferably the composition is in liquid form. The composition may be as defined herein. The composition may further exhibit whitening activity and may be used to whiten teeth.

Further provided is a method of inhibiting bacterial attachment to an oral cavity and/or in reducing bacterial viability in an oral cavity, comprising administering or applying a composition to the oral cavity, wherein the composition is preferably as defined herein.

The composition of the invention is particularly effective against common oral pathogens such as *Actinomyces viscosus, Lactobacillus casei, Fusobacterium nucleatum* subsp. *Polymorphum, Streptococcus oralis* and *Veillonella parvula.* In light of the ant-microbial effects described above, compositions according to the present invention are useful for reducing plaque, and for preventing or treating gingivitis and other forms of periodontal disease. In some embodiments, the compositions are used to prevent or treat gingivitis in conjunction with providing a teeth whitening effect.

In another arrangement, provided herein is a use of an N^{α}-acyl-L-arginme alkyl ester salt in an aqueous mouthwash composition comprising a peroxide source, for maintaining stability of the peroxide source and/or the N^{α}-acyl-L-arginine alkyl ester salt in the composition. Typically, the N^{α}-acyl-L-arginine alkyl ester salt is represented by Formula 1: 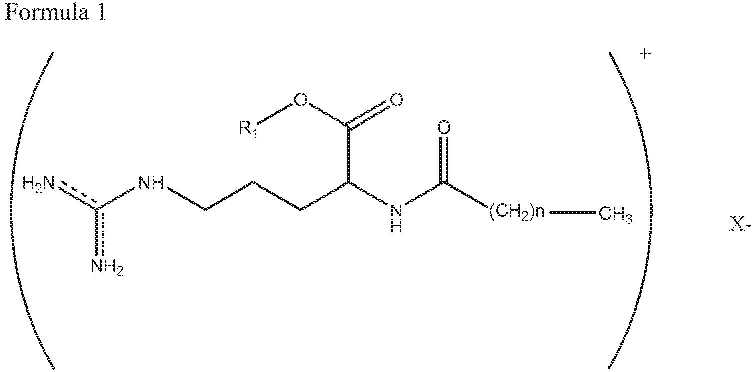 wherein R₁ is an alkyl group having 1 to 6 carbon atoms, n is an integer from 6 to 16, and X⁻ is an anion.

In yet another arrangement, provided herein is a use of a peroxide source in an aqueous mouthwash composition comprising an N^{α}-acyl-L-arginine alkyl ester salt, for maintaining stability of the N^{α}-acyl-L-arginine alkyl ester salt and/or peroxide source in the composition. Typically, the N^{α}-acyl-L-arginine alkyl ester salt is represented by Formula 1: 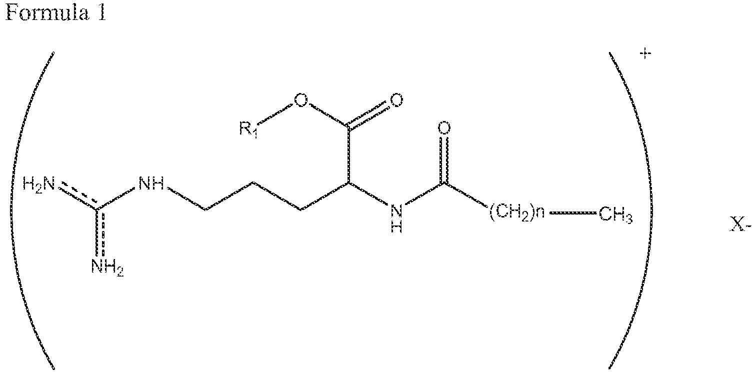 wherein R₁ is an alkyl group having 1 to 6 carbon atoms, n is an integer from 6 to 16, and X⁻ is an anion.

The present inventors have unexpectedly found that when an N^{α}-acyl-L-arginine alkyl ester salt, preferably according to Formula 1, and a peroxide source are combined in an aqueous mouthwash or a mouthrinse formulation, the stability of both agents is maintained.

The following examples illustrate compositions of the invention and their uses. The exemplified compositions are illustrative and do not limit the scope of the invention.

### EXAMPLES

### Example 1 - Stability tests

The stability of ethyl lauroyl arginate hydrochloride (ELA) and hydrogen peroxide (R₂O₂) in a mouthwash formulation comprising ELA and H₂O₂ was assessed. A prototype mouthwash formulation comprising 0.2 weight % ELA on an active ingredient basis (Aminat®-G and 2 weight % H₂O₂ was placed in a stability chamber at 25°C/60% humidity or at 45°C/75% humidity. The stability of H₂O₂ was assessed over a period of eight weeks by measuring oxygen release over a period of eight weeks (H₂O₂ decomposes to hydrogen and oxygen). The stability of ELA was assessed by measuring active ELA concentration over the same period. A mouthwash formulation comprising cetyl pyridinium chloride (CPC) and H₂O₂ was used as a positive control for the experiment (it is known that CPC and H₂O₂ can be combined in mouthwash formulations without impacting on the stability of either agent), and a mouthwash formulation comprising ELA without H₂O₂ was used as a negative control for the experiment. The formulations used in the tests are indicated in Table 1 and the results are indicated in Tables 2 to 6.

**Table 1 - Formulations tested**

| | **ELA w/o H₂O₂** | **ELA w/ H₂O₂** | **CPC w/ H₂O₂** |
|---|---|---|---|
| DEMIN. WATER | 82.26 | 76.55 | 77.45 |
| POLOXOMER 407 | 1.00 | 1.00 | 1.00 |
| SODIUM SACCHARIN | 0.03 | 0.03 | 0.03 |
| Ethyl Lauroyl Arginate HCl | 1.00 | 1.00 | 0.00 |
| CETYLPYRI DINIUM CHLORIDE | 0.00 | 0.00 | 0.10 |
| POLYSORBATE 20 | 0.20 | 0.20 | 0.20 |
| GLYCERIN | 10.00 | 10.00 | 10.00 |
| SORBITOL | 5.50 | 5.50 | 5.50 |
| SUCRALOSE | 0.01 | 0.01 | 0.01 |
| HYDROGEN PEROXIDE (35%) | 0.00 | 5.71 | 5.71 |
| Total (%) | 100.00 | 100.00 | 100.00 |

### Results:

**Table 2 - Stability of ELA at 25°C**

| | ELA (%) at 25°C | | |
|---|---|---|---|
| Formulation | 0 weeks | 4 weeks | 8 weeks |
| ELA w/o H₂O₂ | 0.2 | 0.2 | 0.19 |
| ELA w/ H₂O₂ | 0.19 | 0.19 | 0.19 |

**Table 3 - Stability of ELA at 40°C**

| | ELA (%) at 40°C | |
|---|---|---|
| Formulation | 4 weeks | 8 weeks |
| ELA w/o H₂O₂ | 0.2 | 0.19 |
| ELA w/ H₂O₂ | 0.19 | 0.17 |

**Table 4 - Stability of H₂O₂ at 25°C**

| | Active oxygen (%) at 25°C | | |
|---|---|---|---|
| Formulation | 0 weeks | 4 weeks | 8 weeks |
| ELA w/ H₂O₂ | 1.99 | 1.99 | 2 |
| CPC w/ H₂O₂ | 1.99 | 1.99 | 2 |

**Table 5 - Stability of H₂O₂ at 40°C**

| | Active oxygen (%) at 40°C | |
|---|---|---|
| Formulation | 4 weeks | 8 weeks |
| ELA w/ H₂O₂ | 1.98 | 2 |
| CPC w/ H₂O₂ | 1.98 | 1.99 |

It can be seen from Tables 2 and 3, that the stability of ELA is unexpectedly maintained in the mouthwash formulations of the present invention comprising ELA and H₂O₂, both at 25°C and at 40°C, over a period of 8 weeks. Similarly, Tables 4 and 5 indicate that there was negligible decomposition of H₂O₂ in the presence of ELA and CPC both at 25°C and at 40°C, over a period of 8 weeks. These results demonstrate that ELA and H₂O₂ can be combined within a mouthwash formulation without impacting on the stability of either agent.

### Example 2-48 hour static anti-attachment assay

The anti-microbial efficacy of mouthwash formulations according to the present invention was tested using an anti-attachment assay. The formulations tested were comparable to those provided in Table 1, and comprised the following ELA/ H₂O₂ combinations:
1. 0.2 weight % ELA and 2 weight % H₂O₂
2. 0.2 weight % ELA and 0 weight % H₂O₂
3. 0 weight % ELA and 2 weight % H₂O₂

Hydroxyapatite (HAP) discs were soaked overnight in clarified saliva at 37°C prior to treating for 30 minutes with 1ml of mouthwash formulation. After treatment, the discs were rinsed several times in 1ml DI water. The rinsed discs were subsequently placed in 1ml of an "Artificial mouth" bacterial mix (*A.naeslundii*, *S*. *oralis, V. parvula, L. casei and F. nucleatum*) at an optical density of 0.2 at 610nm, and incubated at 37°C for 48 hours. After incubation, the the discs were treated with trypsin to harvest the biofilm formed on the discs, and the biofilm was quantified by measuring the absorbance at 610nm. The percentage reduction in biofilm formation relative to the untreated discs was calculated. The results are presented in Table 7.

**Table 7 - results of anti-attachment assay**

| | MW 0.2% ELA + 2% H₂O₂ | MW 0.2% ELA + 0% H₂O₂ | MW 0% ELA + 2% H₂O₂ | Total MW 0.075% CPC | No Treatment |
|---|---|---|---|---|---|
| Mean | 0.1398 | 0.1318 | 0.1758 | 0.1398 | 0.2824 |
| St. Dev. | 0.0165 | 0.0136 | 0.0309 | 0.0065 | 0.0129 |
| % reduction | 50.50 | 53.33 | 37.75 | 50.50 | 0.00 |

It can be seen from Table 7 that a mouthwash formulation according to the present invention comprising ELA and H₂O₂ performed comparably to a mouthwash formulation comprising ELA alone, to a mouthwash formulation comprising CPC. These results demonstrate that ELA and H₂O₂ can be effectively combined in a mouthwash formulation to provide whitening effects arising from H₂O₂ (it can be seen from Tables 4 and 5 that stability of H₂O₂ is maintained in the presence of ELA), as well as anti-attachment effects.

### Example 3 - Short Interval Kill test

The anti-microbial efficacy of mouthwash formulations according to the present invention was tested using short interval kill (SIK) test. The formulations tested were the same as those used in Example 2.

500 µl of a mixed-species bacterial culture (*Actinomyces viscosus* (ATCC#43146), *Lactobacillus casei* (ATCC#334), *Fusobacterium nucleatum* subsp. *polymorphum* (ATCC#10953), *Streptococcus oralis* (ATCC#35037) and *Veillonella parvula* (ATCC#17745)) were transferred to sterile 2 ml microcentrifuge tubes. The samples were centrifuged for 10 min at ∼21 000 x g to pellet bacterial cells. The supernatants were aspirated and the pelleted cells were re-suspended in 100 µl of sterile phosphate buffered saline (PBS). The re-suspended samples were subsequently treated with 100 µl of mouthwash or control solution. Bacterial killing was stopped after thirty seconds by adding 1.3 ml of D/E Neutralization Buffer. Samples were centrifuged for 10 min at 10,000 rpm to pellet bacterial cells. The pellets were re-suspended in 500 µl of sterile PBS to wash them, and then centrifuged again. Finally, the pelleted cells were suspended in 150 µl of sterile PBS and aliquots were transferred to each of three wells of a sterile 96-well plate.

To quantify the viable bacteria left after treatment, the bacterial suspensions were stained using Invitrogen BacLight Live/Dead viability kit. This kit consists of two fluorescent dyes, propidium iodine and SYTO9. The first dye, propidium iodide, is a red fluorescent DNA stain that can only penetrate bacterial cells whose membranes have been permeabilized (i.e. those cells that are "dead"). The second dye, SYT09, is a green fluorescent DNA dye that is able to stain all bacterial cells. The bacterial suspensions were stained by adding 50 µl of a solution containing the two dyes in equal amounts to each well of the 96-well plate. Plates were then incubated for 15 min at room temperature, protected from light. The fluorescence of the samples were measured at an excitation wavelength of 485 nm and emission wavelengths of 535 nm and 635 nm. The results are illustrated in Table 8, and are provided as the percentage of cells that are viable relative to a control treatment with PBS alone.

**Table 8 - results of SIK test**

| | Run 1 | Run 2 | Run 3 | Average | St.Dev |
|---|---|---|---|---|---|
| PBS | 100 | 100 | 100 | 100 | 0 |
| Ethanol 100% | 44 | 47 | 48 | 46 | 2.3731 |
| MW 0.2% ELA + 2% H₂O₂ | 55 | 57 | 56 | 56 | 0.6381 |
| MW 0.2% ELA + 0% H₂O₂ | 59 | 63 | 67 | 63 | 3.7125 |
| MW 0% ELA + 2% H₂O₂ | 91 | 94 | 96 | 94 | 2.8577 |
| Total MW 0.075% CPC | 54 | 54 | 59 | 55 | 2.7880 |

As can be seen from Table 8, the formulation according to the present invention comprising ELA and H₂O₂ was most effective in killing bacteria, at a level comparable to the positive control formulation comprising CPC, and a control formulation comprising ELA alone. The bactericidal effect of the formulation of the present invention was significantly more pronounced than that of a formulation comprising H₂O₂ alone in the absence of ELA. These results demonstrate that the bactericidal effects of ELA are not compromised in the presence of H₂O₂, and that ELA may be combined with H₂O₂ in a mouthwash formulation to provide both bactericidal and whitening effects.

## Claims

1. An aqueous mouthwash composition comprising:
(a) a peroxide source, and
(b) an N^{α}-acyl-L-arginine alkyl ester salt.

2. The composition according to claim 1, wherein the N^{α}-acyl-L-arginine alkyl ester salt is represented by Formula 1, 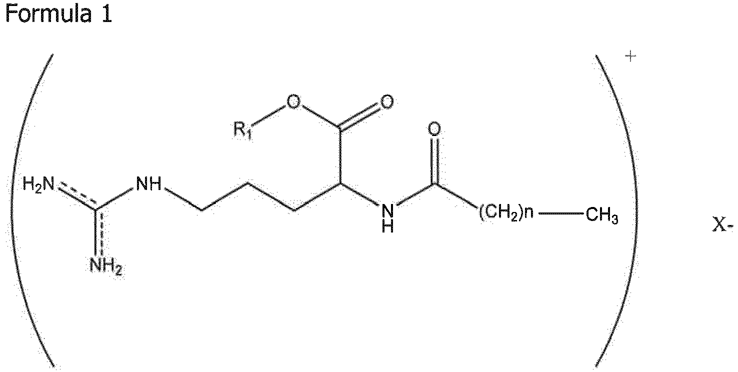 wherein R₁ is an alkyl group having 1 to 6 carbon atoms, n is an integer from 6 to 16, and X- is an anion.

3. The composition according to claim 1 or claim 2, wherein the peroxide source is selected from: hydrogen peroxide, urea peroxide, sodium percarbonate, sodium perborate, polyvinylpyrrolidone-hydrogen peroxide complex, and mixtures thereof.

4. The composition according to any of claims 1 to 3, wherein the peroxide source is present in the composition in an amount of 0.1 weight % to 3 weight %, preferably 1 weight % to 2 weight %, further preferred 2 weight %, by total weight of the composition.

5. The composition according to any of claims 2 to 4, wherein R₁ is ethyl and n is 10, preferably
wherein the N^{α}-acyl-L-arginine alkyl ester salt comprises ethyl lauroyl arginine hydrochloride.

6. The composition according to any preceding claim, wherein the N^{α}-acyl-L-arginine alkyl ester salt is present in the composition in an amount of from 0.05 weight % to 0.4 weight %, preferably 0.1 weight % to 0.3 weight, further preferred 0.2 weight %, by total weight of the composition, on an active ingredient basis.

7. The composition according to any preceding claim, wherein the composition further comprises an agent selected from surfactants, humectants, foaming agents, flavorants and sweeteners, preferably
wherein the composition further comprises a non-ionic surfactant, further preferred wherein the non-ionic surfactant is selected from poloxamers and polyoxyethylene sorbitan esters.

8. The composition according claim 7 comprising a poloxamer and a polyoxyethylene sorbitan ester.

9. The composition according to claim 7 or 8, wherein the composition further comprises a humectant, preferably
wherein the composition comprises glycerin and/or sorbitol.

10. The composition according to any preceding claim, wherein the composition comprises water in an amount of 70 weight % to 80 weight %, by total weight of the composition,and/or wherein
wherein the composition is free of anti-microbial agents other than the N^{α}-acyl-L-arginine ethyl ester salt and the peroxide source.

11. An aqueous mouthwash composition comprising:
(a) a peroxide source, and
(b) an N^{α}-acyl-L-arginine alkyl ester salt;
for use in inhibiting bacterial attachment to an oral cavity and/or in reducing bacterial viability in an oral cavity.

12. The composition for use according to claim 11, wherein the use further comprises whitening teeth, and/or
wherein the use comprises preventing or treating gingivitis.

13. The composition for use according to claim 11 or 12, wherein the composition is as defined in any of claims 2 to 10.

14. Use of an N^{α}-acyl-L-arginine alkyl ester salt in an aqueous mouthwash composition comprising a peroxide source, for maintaining stability of the peroxide source.

15. The use according to claim 14, wherein the composition is as defined in any of claims 2 to 10.

## Patentansprüche

1. Wässrige Mundwasserzusammensetzung, umfassend:
(a) eine Peroxidquelle, und
(b) ein N^{α}-Acyl-L-arginin-alkylestersalz.

2. Zusammensetzung nach Anspruch 1, wobei das N^{α}-Acyl-L-arginin-alkylestersalz durch die Formel 1 dargestellt ist, 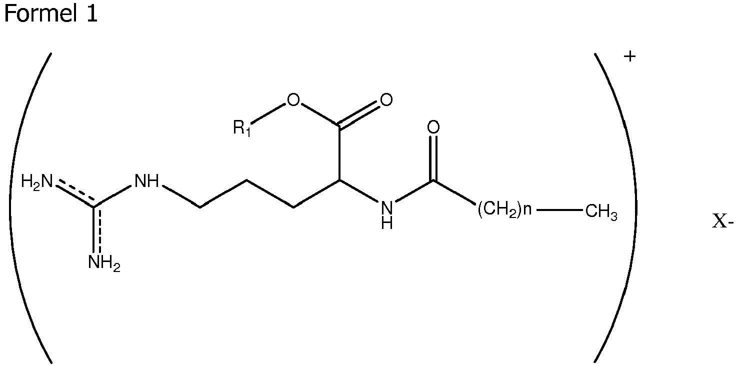 wobei R₁ eine Alkylgruppe mit 1 bis 6 Kohlenstoffatomen ist, n eine Ganzzahl von 6 bis 16 ist, und X- ein Anion ist.

3. Zusammensetzung nach Anspruch 1 oder Anspruch 2, wobei die Peroxidquelle ausgewählt ist aus: Wasserstoffperoxid, Harnstoffperoxid, Natriumpercarbonat, Natriumperborat, Polyvinylpyrrolidon-Wasserstoffperoxidkomplex, und Gemischen davon.

4. Zusammensetzung nach einem beliebigen der Ansprüche 1 bis 3, wobei die Peroxidquelle in der Zusammensetzung in einer Menge von 0,1 Gew.-% bis 3 Gew.-%, bevorzugt 1 Gew.-% bis 2 Gew.-%, weiter bevorzugt 2 Gew.-%, bezogen auf das Gesamtgewicht der Zusammensetzung vorliegt.

5. Zusammensetzung nach einem beliebigen der Ansprüche 2 bis 4, wobei R₁ Ethyl ist und n 10 ist, bevorzugt
wobei das N^{α}-Acyl-L-arginin-alkylestersalz Ethyllauroylarginin-hydrochlorid umfasst.

6. Zusammensetzung nach einem beliebigen voranstehenden Anspruch, wobei das N^{α}-Acyl-L-arginin-alkylestersalz in der Zusammensetzung in einer Menge von 0,05 Gew.-% bis 0,4 Gew.-%, bevorzugt 0,1 Gew.-% bis 0,3 Gew.-%, weiter bevorzugt 0,2 Gew.-%, bezogen auf das Gesamtgewicht der Zusammensetzung auf einer aktiven Wirkstoffbasis vorliegt.

7. Zusammensetzung nach einem beliebigen voranstehenden Anspruch, wobei die Zusammensetzung des Weiteren ein Mittel umfasst, das aus oberflächenaktiven Mitteln, Feuchthaltemitteln, Schaummitteln, Aromastoffen und Süßstoffen ausgewählt ist, bevorzugt
wobei die Zusammensetzung des Weiteren ein nicht ionisches oberflächenaktives Mittel umfasst, des Weiteren bevorzugt
wobei das nicht ionische oberflächenaktive Mittel aus Poloxameren und Polyoxyethylensorbitanestern ausgewählt ist.

8. Zusammensetzung nach Anspruch 7, die ein Poloxamer und einen Polyoxyethylensorbitanester umfasst.

9. Zusammensetzung nach Anspruch 7 oder 8, wobei die Zusammensetzung des Weiteren ein Feuchthaltemittel umfasst, bevorzugt
wobei die Zusammensetzung Glycerin und/oder Sorbitol umfasst.

10. Zusammensetzung nach einem beliebigen voranstehenden Anspruch, wobei die Zusammensetzung Wasser in einer Menge von 70 Gew.-% bis 80 Gew.-%, bezogen auf das Gesamtgewicht der Zusammensetzung umfasst, und/oder wobei die Zusammensetzung frei von antimikrobiellen Mitteln außer dem N^{α}-Acyl-L-arginin-ethylestersalz und der Peroxidquelle ist.

11. Wässrige Mundwasserzusammensetzung, umfassend:
(a) eine Peroxidquelle, und
(b) ein N^{α}-Acyl-L-arginin-alkylestersalz;
zur Verwendung beim Inhibieren von bakteriellem Anheften an eine Mundhöhle und/oder beim Verringern von bakterieller Viabilität in einer Mundhöhle.

12. Zusammensetzung zur Verwendung nach Anspruch 11, wobei die Verwendung des Weiteren Aufhellen von Zähnen umfasst, und/oder
wobei die Verwendung Vorbeugen oder Behandeln von Gingivitis umfasst.

13. Zusammensetzung zur Verwendung nach Anspruch 11 oder 12, wobei die Zusammensetzung wie in einem beliebigen der Ansprüche 2 bis 10 definiert ist.

14. Verwendung eines N^{α}-Acyl-L-arginin-alkylestersalzes in einer wässrigen Mundwasserzusammensetzung, die eine Peroxidquelle umfasst, zum Beibehalten der Stabilität der Peroxidquelle.

15. Verwendung nach Anspruch 14, wobei die Zusammensetzung wie in einem beliebigen der Ansprüche 2 bis 10 definiert ist.

## Revendications

1. Composition aqueuse pour bain de bouche comprenant :
(a) une source de peroxyde, et
(b) un sel d'ester alkylique de N^{α}-acyl-L-arginine.

2. Composition selon la revendication 1, dans laquelle le sel d'ester alkylique de N^{α}-acyl-L-arginine est représenté par la formule 1, 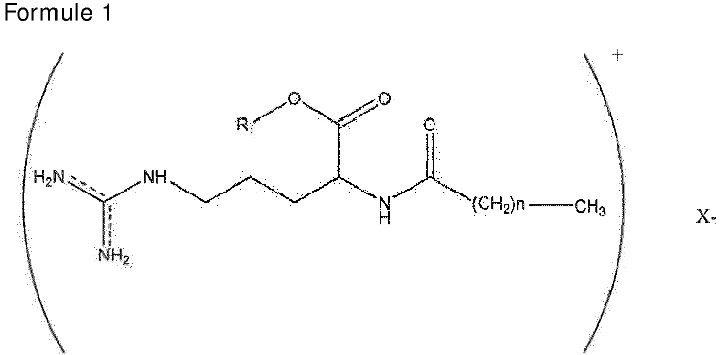 dans laquelle R₁ est un groupe alkyle ayant 1 à 6 atomes de carbone, n est un nombre entier de 6 à 16, et X- est un anion.

3. Composition selon la revendication 1 ou la revendication 2, dans laquelle la source de peroxyde est choisie parmi : le peroxyde d'hydrogène, le peroxyde d'urée, le percarbonate de sodium, le perborate de sodium, un complexe de polyvinylpyrrolidone-peroxyde d'hydrogène, et leurs mélanges.

4. Composition selon l'une quelconque des revendications 1 à 3, dans laquelle la source de peroxyde est présente dans la composition en une quantité de 0,1 % en poids à 3 % en poids, de préférence de 1 % en poids à 2 % en poids, de manière davantage préférée de 2 % en poids, en poids total de la composition.

5. Composition selon l'une quelconque des revendications 2 à 4, dans laquelle R₁ est un groupe éthyle et n est 10, de préférence
dans laquelle le sel d'ester alkylique de N^{α}-acyl-L-arginine comprend le chlorhydrate d'éthyl lauroyl arginine.

6. Composition selon l'une quelconque des revendications précédentes, dans laquelle le sel d'ester alkylique de N^{α}-acyl-L-arginine est présent dans la composition en une quantité de 0,05 % en poids à 0,4 % en poids, de préférence de 0,1 % en poids à 0,3 % en poids, de manière davantage préférée de 0,2 % en poids, en poids total de la composition, sur la base du principe actif.

7. Composition selon l'une quelconque des revendications précédentes, dans laquelle la composition comprend en outre un agent choisi parmi des tensioactifs, des humectants, des agents moussants, des aromatisants et des édulcorants, de préférence
dans laquelle la composition comprend en outre un tensioactif non ionique, de manière davantage préférée
dans laquelle le tensioactif non ionique est choisi parmi les poloxamères et les esters de sorbitan polyoxyéthylénés.

8. Composition selon la revendication 7, comprenant un poloxamère et un ester de sorbitan polyoxyéthyléné.

9. Composition selon la revendication 7 ou 8, dans laquelle la composition comprend en outre un humectant, de préférence
dans laquelle la composition comprend de la glycérine et/ou du sorbitol.

10. Composition selon l'une quelconque des revendications précédentes, dans laquelle la composition comprend de l'eau en une quantité de 70 % en poids à 80 % en poids, en poids total de la composition, et/ou dans laquelle
dans laquelle la composition est exempte d'agents antimicrobiens autres que le sel d'ester éthylique de N^{α}-acyl-L-arginine et la source de peroxyde.

11. Composition aqueuse pour bain de bouche comprenant :
(a) une source de peroxyde, et
(b) un sel d'ester alkylique de N^{α}-acyl-L-arginine ;
pour une utilisation dans l'inhibition de la fixation bactérienne à une cavité buccale et/ou dans la réduction de la viabilité bactérienne dans une cavité buccale.

12. Composition pour une utilisation selon la revendication 11, dans laquelle l'utilisation comprend en outre le blanchiment des dents, et/ou
dans laquelle l'utilisation comprend la prévention ou le traitement de la gingivite.

13. Composition pour une utilisation selon la revendication 11 ou 12, dans laquelle la composition est telle que définie dans l'une quelconque des revendications 2 à 10.

14. Utilisation d'un sel d'ester alkylique de N^{α}-acyl-L-arginine dans une composition aqueuse pour bain de bouche comprenant une source de peroxyde, afin de maintenir la stabilité de la source de peroxyde.

15. Utilisation selon la revendication 14, dans laquelle la composition est telle que définie dans l'une quelconque des revendications 2 à 10.
